# EUROPEAN PATENT SPECIFICATION

(11) **EP 1 986 594 B1**
(45) Date of publication and mention of the grant of the patent: **27.09.2017**
(21) Application number: 07762679.4
(22) Date of filing: 26.01.2007
(51) Int. Cl.: A61K 8/26, A61Q 3/02, B82Y 5/00

(54) **COATINGS FOR MAMMALIAN NAILS THAT INCLUDE NANOSIZED PARTICLES**
BESCHICHTUNGEN FÜR SÄUGETIERNÄGEL MIT PARTIKELN IN NANOGRÖSSE
REVÊTEMENTS POUR ONGLES DE MAMMIFÈRES COMPRENANT DES NANOPARTICULES

(30) Priority: 27.01.2006 US 743180 P
(43) Date of publication of application: 05.11.2008
(73) Proprietor: Coty S.A.S., 75002 Paris (FR)
(72) Inventor: AMATO, Steven, W., North Plainfield, NJ 07060 (US); FARER, Alan, Kinnelon, NJ 07405 (US); HOYTE, Wayne, M., Parlin, NJ 08859 (US); PAVLOVSKY, Michael, Parsippany, NJ 07054 (US); SMITH, Raymond, Verona, NJ 07044 (US); VALDIVIEZO, Grisel, Parsippany, NJ 07054 (US)
(74) Representative: Hanna Moore + Curley
(86) International application number: PCT/US2007/002207
(87) International publication number: WO 2007/089604

(56) References cited:
- US-A- 5 290 543
- US-A1- 2003 064 086
- US-A1- 2004 223 990
- US-A1- 2005 220 730
- US-A1- 2006 002 875
- US-A1- 2006 002 875
- US-B1- 6 627 181
- Anonymous: "Nanomer I.34TCN", , XP002693506, Retrieved from the Internet: URL:http://www.east8west.cn/data/%E6%9C%89 %E6%9C%BA%E8%92%99%E8%84%B1%E5%9C%9F-I.34T CN.pdf [retrieved on 2013-03-07]
- Anonymous: "Cloisite Additives", , 2013, XP002693507, Retrieved from the Internet: URL:http://www.nanoclay.com/benefits2.asp [retrieved on 2013-03-07]
- anonymous: "Sodium bentonite: its structure and properties", , 2013, Retrieved from the Internet: URL:http://www.cetco.com/DesktopModules/Br ing2mind/DMX/Download.aspx?Command=Core_Do wnload&PortalId=0&EntryId=538 [retrieved on 2015-11-27]
- ANONYMOUS: 'Particle size', [Online] Retrieved from the Internet: <URL:https://en.wikipedia.org/wiki/Particle _size> [retrieved on 2015-12-09]
- anonymous: "A guidebook to particle size analysis", , 2014, Retrieved from the Internet: URL:https://www.horiba.com/fileadmin/uploa ds/Scientific/Documents/PSA/PSA_Guidebook. pdf [retrieved on 2015-12-09]
- anonymous: "Nanoclay structures", , 2008, Retrieved from the Internet: URL:http://www.nanocor.com/nano_struct.asp [retrieved on 2015-11-27]

## Description

### Field

Embodiments of the invention described relate to coatings for mammalian nails that include nanosized particles and methods for making the coatings.

### Copyright

A portion of the disclosure of this patent document contains material that is subject to copyright protection. The copyright owner has no objection to the facsimile reproduction by anyone of the patent document or the patent disclosure, as it appears in the Patent and Trademark Office patent files or records, but otherwise reserves all copyright rights whatsoever. The following notice applies to the products, processes and data as described below and in the tables that form a part of this document: Copyright 2006, Coty Inc. All Rights Reserved.

### Background

There are many commercial nail enamel products on the market that include traditional clay compounds such as Stearalkonium Hectorite, Disteardimonium Hectorite, Quaternium-18 Bentonite, Stearalkonium Bentonite, and silica as a rheological additive. When these clays are modified with a cation, such as a quaternary ammonium cation, the modified clays, when processed appropriately, form a gel having thixotropic properties. The gel prevents sedimentation of pigment in nail enamel formulations.

US Patent Application No. US 2004/223990A1 describes a physiologically acceptable medium containing at least one fatty phase and exfoliated phyllosilicates derived from at least one phyllosilicate intercalated with a non-polyphenolic intercalating agent.

US Patent No. US 6,627,181 (B1) discloses an enamel base coat layer and a nail repair and adhesive layer contained in a two layered solution in a single nail polish type bottle. The nail repairing and strengthening layer is the lower layer which has both higher viscosity and higher specific gravity than the upper layer.

US Patent Application No. US 2005/220730 (A1) discloses a nail polish composition which comprises about 0.01% to 20% by weight of non-aggregated particles which have a mean size between about 5 nm and about 100 nm, a main film forming polymer, a secondary film forming polymer, at least one plasticizer, at least one solvent, wherein the particles do not have reactive groups on a surface, and wherein the particles have a refractive index between about 1.4 and about 1.7.

US Patent Application No. US 2006/002875 (A1) relates to the use of diffractive colorants in cosmetics, to compositions comprising diffractive colorants, and to processes for the preparation of the compositions and to the use thereof.

### Description

Although detailed embodiments of the invention are disclosed herein, it is to be understood that the disclosed embodiments are merely exemplary of the invention that may be embodied in various and alternative forms. Specific structural and functional details disclosed herein are not to be interpreted as limiting, but merely as a basis for teaching one skilled in the art to variously employ the nail enamel embodiments. Throughout the drawings, like elements are given like numerals.

Referred to herein are trade names for materials including, but not limited to, polymers and optional components. The inventors herein do not intend to be limited by materials described and referenced by a certain trade name. Equivalent materials (e.g., those obtained from a different source under a different name or catalog (reference) number to those referenced by trade name may be substituted and utilized in the methods described and claimed herein. All percentages and ratios are calculated by weight unless otherwise indicated. All percentages are calculated based on the total composition unless otherwise indicated. All component or composition levels are in reference to the active level of that component or composition, and are exclusive of impurities, for example, residual solvents or by-products, which may be present in commercially available sources.

Mammalian nail coating composition embodiments of the invention include nanopigments and nanoclays; and, optionally, nanomaterials such as nanosilica, nanoceramics and mixtures of these nanomaterials as rheological additives heretofore unknown for use in nail enamel compositions. The mammalian nail coatings that include one or more of these nanoparticle materials have desirable performance attributes that include increased glossiness, smoothness, and surface slip. For some embodiments of the nail enamel compositions, the amount of Nano- Sized Clays, silica, pigments and ceramics for use range from about 0.10 to 10.0% by weight. For some composition embodiments, the range of nanoparticle concentration is about 0.5 to 5.0 by weight.

In its product aspect, embodiments of the present invention provide nail enamel compositions which are suitable for use as base coats, color coats, clear coats and protective top coats while maintaining or surpassing the desirable characteristics of the nail enamel compositions. The nail enamel compositions, when applied to nails, impart increased toughness, increased strength, increased impact resistance, increased mar resistance, increased flexibility and increased wear properties. As used herein, the terms "lacquer," "polish" and "enamel" are used interchangeably.
Rheological nanosized additives, by themselves or in combinations with other Rheological Additives such as Stearalkonium Hectorite-, Disteardimonium Hectorite, Quaternium-18 Bentonite, Stearalkonium Bentonite or silica, in composition embodiments described herein are in amounts sufficient to provide significantly improved physical properties to a nail enamel film on natural or synthetic nail surfaces. Specific examples of specific Bentonite include, but are not limited to Bentone 2004, and Bentone 166, alkylaryl ammonium hectorite, both manufactured by Elementis Specialties of Hightstown, New Jersey.

While nanoclay and nanopigments are described herein, it is understood that other nail enamel compositions that also include nanosilica and nanoceramic particles, and combinations of these nanoparticles are also suitable for use. The nanoclay exhibits many unique properties, in part, because the particle size is less than the wavelength of visible light. This means that light passes between these very fine platelets. The results include nail enamels that have a greater gloss and depth of color.

For some embodiments, the nanoclay raw material is a particle having a 1- to 2-layer clay mineral with a platelet structure. Multilayer structures are also within the scope of invention embodiments. Individual platelet thicknesses are just one nanometer, (one-billionth of a meter), but surface dimensions are generally 70 to more than 150 nanometers, resulting in an unusually high aspect ratio. These materials are very fine platelets that are roughly 1/10,000th the diameter of a human hair in thickness.

When the nanoclay is dispersed in traditional nail polish or polymers, the nanoclay delivers a unique combination of performance and processing advantages including smoothness and toughness. When the nail polish is applied to the nail, a greater portion of the nanoclay settles into the natural striations of the nail. This allows for a tighter bond to the nail and a smoother surface when the nail polish is dry. These features of smoothness and toughness are manifested by greater shock resistance, more stretchability and flexibility.

The composition embodiments described herein, may be prepared by a sequential addition of all the ingredients or through several steps. For example, in the case of some mammalian nail coating embodiments, the nanoparticles may be mixed in a thixotrope such as a bentone gel. However, the nanoclays themselves form gels similar to their larger particle size counterparts. Processing parameters may need to be changed in manners well known to those skilled in the art.

In one embodiment, the nanoparticles are coated nanoparticles. The nanoparticles are coated with an ammonium-based material such as dimethyl, dehydrogenated tallow, quaternary ammonium anion, where the anion is, for some embodiments, chloride. The hydrogenated tallow is about 65% C(10), 30% C(16), and about 5% C(14). For some embodiments, the nanoparticles are a natural montmorillonite modified with a quaternary ammonium salt. This nanoparticle embodiment is obtained from Southern Clay Products, Inc., of Gonzales, Texas, and are known as Cloisite ® 15A. Another nanoparticle embodiment is an organically modified bentonite clay, Bentone 1651, manufactured by Elementis Specialties or Hightstown, New Jersey. Other surface treatments for the nanoparticles include blending with one or more of the following: siloxane star-graft polymer coating; epoxy functional silane; tetra-functional silane; carboxylic acid; and anhydride. For some embodiments, nanoparticles that have been coated with an ammonium salt or hydroxide are further coated with an organic material such as hexylamine, dodecylamine, trioxtylmethylammonium, tridodecilmethylammonium and similar organic material.

Prior to incorporation of the nanoparticles into the nail enamel formulation, the nanoparticles may be deagglomerated and stabilized. For some embodiments, ultrasound is used for this application. A liquid jet stream resulting from ultrasonic cavitation, overcomes the bonding forces, e.g. van der Waals' forces between the nanoparaticles, and deagglomerates the nanoparticles. Because of the ultrasonically generated shear forces and micro turbulences, ultrasound may assist in the surface coating and chemical reaction of nanoparticles with other materials. For other embodiments, homogenizers and rotor-stator mixers are usable to deagglomerate the nanoparticles.

Other ingredients used in the nail enamel formulations include film-forming polymers. The film-forming polymers used in the nail enamel embodiments described herein are selected from polyurethanes, polyacryls, polymethacryls, cellulosic polymers, styrene-acryl copolymers, polystyrene-polyacryl mixtures, polysiloxanes, polyethers, polyesters, urethane-acryl copolymers, cellulose acetate propionate, siloxane-urethane copolymers, polyurethane-polymethacryl mixtures, silicone-acryl copolymers, vinyl acetate polymers, and mixtures thereof. Exemplary secondary film forming resins usable in the formulations of the present invention include, for example, toluene sulfonamide/epoxy resins, e.g., tosylamide and non-drying alkyd resins, acrylic polymers and copolymers, polyurethane, polyacryls, polymethacryls, cellulosic polymers, styrene- acryl copolymers, polystyrene-polyacryl mixtures, polysiloxanes, polyethers, polyesters, cellulose acetate propionate, urethane-acryl copolymers, siloxane-urethane copolymers, polyurethane-polymethacryl mixtures, polyurethane urea polymers, silicone-acryl copolymers, vinyl acetate polymers, and mixtures thereof.

It is also within the scope of the nail enamel compositions to include aldehyde condensation products such as arylsulfonamide formaldehyde resins, specifically toluene sulfonamide formaldehyde resin which is a condensation product of formaldehyde and toluene sulfonamide. These secondary film forming resins are added to the nail enamel composition embodiments to strengthen and add acceptable wear characteristics to the primary film forming polymer. In general, the amount of secondary film forming resin ranges form about 2 to 20 percent by weight of the composition, and, for some embodiments, about 5 to 12 percent of the composition.

Nitrocellulose is a primary film-forming component in some formulation embodiments described herein. A use of low levels of nitrocellulose tends to result in the coated films being easily damaged. On the other hand, a use of high levels of nitrocellulose results in the coated film being too hard and inflexible, resulting in undesirable peeling and hence poor wear resistance.

Other satisfactory film-forming components include cellulose acetate, cellulose acetate butyrate, cellulose acetate propionate, vinyl polymers, polyurethanes, and mixtures of polyurethanes with cellulose acetate butyrate or with nitrocellulose as well as methacrylate and acrylate type polymers. The film-forming component is present in an amount sufficient to provide a stable film upon the nail enamel following the application of the nail enamel.

In general, amounts of the film-forming component of about 8 to about 40 weight percent for some embodiments, and about 10 to about 15 weight percent for other embodiments, are satisfactory. Examples of nitrocellulose are the so called nitrocellulose RS ⅛ sec., RS ¼ sec., and nitrocellulose ½ sec. and nitrocellulose RS 5-6 sec. and 60-80 sec., which have higher viscosities than the earlier grades. The term "RS" refers to the brand of nitrocellulose with a nitrogen content of about 11.2 to 12.8 percent with solubility in esters, ketones, and glycol ethers. The terms ⅛ sec., ½ sec, 5-6 sec, and so forth represent viscosity and refer to the time it takes for a ball to fall a given depth in the material. Nitrocellulose is typically supplied in 70% concentrations, wet with 30% ethyl or isopropyl alcohol. The percentages of nitrocellulose described herein are on a dry basis of composition embodiments. While specific nitrocellulose formulations are described herein, the specific formulations are included as examples and are not meant to limit methods and formulations claimed herein.

In one embodiment, the resins include Shin-Etsu KP- 543 (acrylic-silicone resin), Uniplex 670P (polyester resin), PCI Group Inc. Lacq-Hard LH-75 (polyamide resin). While specific resin formulations are described herein, the specific formulations are included as examples and are not meant to limit methods and formulations claimed herein.

Suitable plasticizers are incorporated in the formulation to provide flexibility while exhibiting the wear and chip resistance and shelf stability needed for the composition. Without intending to be limited by theory, it is believed that plasticizers cause a composition to become more easily deformed. Typically, plasticizers are needed in the composition embodiments of the present invention. The composition embodiments of the present invention may comprise from 0% to about 15%, for some embodiments, from 0% to about 10%, and in some embodiments, from about 0% to about 7%, by weight of the composition, of a plasticizer. One or more plasticizers are added to the composition embodiments.

Usable plasticizers include but are not limited to camphor, castor oil, Unitex Chemical Corporation Uniplex 108 (N-ethyl-o/p- Toluenesulfonamide), Uniplex 125A (di-(2-ethylhexyl) adipate), Uniplex 165 (diisobutyl Adipate), Uniplex 260 (Glyceryl Tribenzoate), Uniplex 552 (Pentaerytritrol tetrabenzoate), Phoenix Chemical Inc. Pelemol DIA (diisopropyl adipate), Pelemol TMEB-35 (Trimethylolethane Tribenzoate), Pelemol PTB-35 (Pentaerythyl Tetrabenzoate), Pelemol GTO (Glyceryl Trioctanoate), Alzo International Dermol DPG-2B (Dipropylene glycol dibenzoate), Dermol B-246 (Benzyl Laurate Myristate/Palmitate), Eastman Chemical Corporation TXIB (2,2,4-trimethyl-1,3-pentanediol diisobutyrate), SAIB (Sucrose acetate isobutyrate), Ferro Santicizer 160(Butyl Benzyl Phthalate), Velsicol Chemical Corporation Benzoflex 284 (Propylene glycol dibenzoate, Dipropylene glycol dibenzoate, propylene glycol monobenzoate) Benzoflex 354 (2,2,4-trimethyl-1,3-pentanediol dibenzoate), Velate 368 (2- ethylhexyl benzoate), CasChem DIPA (diisopropyl adipate).

For some embodiments, plasticizers include Unitex Chemical Corporation Uniplex PX5-115 (N-Alkyl Toluenesulfonamide), Phoenix Chemical Inc. Pelemol B66 (Benzyl Benzoate), Pelemol BIP (Butyl Phthalimide Isopropyl Phthalimide Blend), Eastman Chemical Corporation Triacetin (Glyceryl triacetate), Uniplex 82 Acetyl Tributyl Citrate. While specific plasticizer formulations are described herein, the specific formulations are included as examples and are not meant to limit methods and formulations claimed herein.

The nail enamel composition embodiments include one or more solvents such as those generally used in conventional nail enamel compositions. Examples of these solvents include ethyl acetate, methyl acetate, n-butyl acetate, iso-butyl acetate, propyl acetate, iso-propyl acetate, amyl acetate, ethanol, isopropanol, n-amyl alcohol, n-butyl alcohol, iso-butanol, cellosolve, butyl cellosolve acetate, cellosolve acetate, methyl cellosolve acetate, butyl cellosolve, ethyl cellosolve, n-butanol, xylene, aromatic (containing phenyl groups), ethers, ketones for example acetone, methyl ethyl ketone, alkanes for example, pentane, cyclopentane, hexane, heptane, cyclohexane, cyclic ethers for example, tetrahydrofuran and 1,4-dioxane, phenylated solvents for example, xylene, chlorinated hydrocarbons for example, methylene chloride, chloroform, glycol ethers, N-methyl pyrrolidone alkyl lactate, and methylchloroform. It is also contemplated that toluene, if desired, can be included as a solvent or diluent for use in a nail enamel composition in accordance with the compositions of invention embodiments.

The aforementioned solvents can be used alone or in mixtures thereof. In general, the amount of solvent used in the composition embodiments of the invention range from about 53 to 82% by weight, and, for some embodiments about 65 to 75% by weight. For some embodiments, organic solvents are selected from alcohols and esters having between one and about twenty-five carbon atoms. For some embodiments, alcohols are monohydric. It is also contemplated that, if desired, monohydric alcohols are selected from ethanol, iso-propanol, and n-propanol. For some embodiments, esters are selected from ethyl acetate and butyl acetate. For other embodiments, it is contemplated that polyhydric alcohols are usable.

Presented below are nail coating embodiments of the invention. The examples presented herein are intended to further describe invention embodiments and not to limit them.

### Example 1

One nail coating composition embodiment is as follows:

| | Colored Nail Coating | |
|---|---|---|
| | Ingredient | Weight percent |
| | Ethyl Acetate | 36.78 |
| | Butyl Acetate | 18.19 |
| | Nitrocellulose (dry) | 14.05 |
| | Isopropyl alcohol | 7.80 |
| | Polyester resin | 8.50 |
| | Epoxy Resin | 3.62 |
| | Quaternium-18 Bentonite nanoclay | 1.00 |
| | Acrylic copolymer | 1.17 |
| | Acetyl Tributyl Citrate | 5.45 |
| | D&C Red # 7C Calcium Lake | 0.40 |
| | Mica | 3.04 |
| | | |
| | TOTAL | 100.00 |

Nanoparticles in this formulation include the Quaternium-18 bentonite nanoclay.

### Example 2

Example 2 illustrates a comparison of features of the composition of claim 1 and a conventional long wearing formulation, that does not include nanoparticles.

### COMPARATIVE Evaluation :

| | **Red pearl shade** | | **%Change** |
|---|---|---|---|
| | Conventional | Nano | |
| | | | |
| Average Dry Time (min' sec") on Nails | | | |
| | | | |
| 1 coat | 2'15" | 2'01" | -10.4 |
| 2 coats | 4'59" | 5'05" | +2.00 |
| | | | |
| Hardness | 106 | 97 | -8.5 |
| | | | |
| Flexibility | OK | OK | NA |
| | | | |
| Adhesion (5=best: 1=worst) (cross hatch on glass) | 4 | 5 | +25 |
| | | | |
| Mechanical Cupping Tests | 6.3 | 7.4 | +17.5 |
| | | | |
| Variable Height Impact Tester | 7.5 | 12.5 | +66.7 |
| | | | |
| Gloss (60 deg GM) | 74.2 | 85.23 | +14.9 |

### Example 3

Example 3 describes a formulation that includes two types of clays, a nanoclay of quaternium-18 Bentonite and a clay having particles larger than nanoparticles, stearalkonium hectorite.

| | Colored Nail Coating | |
|---|---|---|
| | Ingredient | Weight percent |
| | Ethyl Acetate | 36.78 |
| | Butyl Acetate | 18.19 |
| | Nitrocellulose (dry) | 14.05 |
| | Isopropyl alcohol | 7.80 |
| | Polyester resin | 8.50 |
| | Epoxy Resin | 3.62 |
| | Quaternium-18 Bentonite nanoclay | 0.50 |
| | Stearalkonium Hectorite | 0.50 |
| | Acrylic copolymer | 1.17 |
| | Acetyl Tributyl Citrate | 5.45 |
| | D&C Red # 7C Calcium Lake | 0.40 |
| | Mica | 3.04 |
| | | |
| | TOTAL | 100.00 |

### Example 4

Presented below is a table showing performance with respect to hardness, flexibility, adhesion, mechanical cupping tests, variable height impact, and gloss for nail enamel formulations that include nanoclay particles (Nano), and nail enamel formulations that include nanoclay particles and a rheological additive, such as stearalkonium hectorite in a 50/50 blend (Nano+) and conventional nail enamel (Current).

### 2. Current Long Wearing Base vs. Nano and Nano+*

| Light | **Light Pink Frost Shade** | | |
|---|---|---|---|
| | Current | Nano | Nano+* |
| Hardness | 113 | 111 | 107 |
| Flexibility | OK | OK | OK |
| Adhesion (5=best: 1=worst) (cross hatch on glass) | 4 | 5 | 5 |
| Mechanical Cupping Tests | 7.0 | 8.0 | 7.5 |
| Variable Height Impact Tester | 7.5 | 12.5 | 15.0 |
| Gloss (60 deg GM) | 81.3 | 89.8 | 89.2 |

| | | | |
|---|---|---|---|
| Nano+*-Nano clay plus Conventional Rheological additive, such as Stearalkonium Hectorite (50/50 blend) | | | |

### The List of Standard Test Methods Used for Nail Enamel Products

| | |
|---|---|
| ASTM D522-939 | Mandrel Bend Test of Attached Organic Coatings |
| ASTM D523-89 | Specular Gloss |
| ASTM D2794-93 | Resistance of Organic coatings for the Effect of Rapid Deformation (Impact) |
| ASTM D4366 | Hardness of Organic Coatings by Pendulum Damping Test |
| DIN EN IS) 1520 | Mechanical Cupping Method for the Measurement of the Elongation of Coatings on Metallic Substrates |

Rheological additives usable with nanoparticles to make the Nano + blend include the following:

| | |
|---|---|
| Stearalkonium Bentonite- | Stearalkonium Bentonite is a Reaction product of Bentonite and Stearalkonium Chloride |
| Quaternium-18 Bentonite - | Quaternium-18 Bentonite is reaction product of Bentonite and Quaternium-18 |
| Disteardimonium Hectorite- | Disteardimonium Hectorite is the reaction product of Distearyldimonium Chloride and Hectorite |
| Stearalkonium Hectorite- | Stearalkonium Hectorite is a reaction product of Hectorite and Stearalkonium Chloride |
| Silica- | Silica is the inorganic oxide that conforms to the formula SiO₂. It functions as a Suspending Agent. This includes both untreated amorphous silica and surface treated silica. |
| Quaternium-18 Bentonite (nanosize)- | Quaternium-18 Bentonite is a reaction product of Bentonite and Quaternium-18. This is the nanoclay that we are using. |

### Example 5

The formulations below are embodiments of a nail enamel formulation that includes a quaternium-18 bentonite nanoclay. The second embodiment includes the Nano + formulation.

| Colored Nail Coating--1 | |
|---|---|
| Ingredient | Weight percent |
| Ethyl Acetate | 36.78 |
| Butyl Acetate | 18.19 |
| Nitrocellulose (dry) | 14.05 |
| Isopropyl alcohol | 7.80 |
| Polyester resin | 8.50 |
| Epoxy Resin | 3.62 |
| Quaternium-18 Bentonite Nanoclay | 0.50 |
| Acrylic copolymer | 1.17 |
| Acetyl Tributyl Citrate | 5.45 |
| D&C Red # 7C Calcium Lake | 0.40 |
| Mica | 3.04 |
| TOTAL | 100.00 |

| 2. | |
|---|---|
| Ingredient | Weight percent |
| Ethyl Acetate | 36.78 |
| Butyl Acetate | 18.19 |
| Nitrocellulose (dry) | 14.05 |
| Isopropyl alcohol | 7.80 |
| Polyester resin | 8.50 |
| Epoxy Resin | 3.62 |
| Quaternium-18 Bentonite Nanoclay | 0.50 |
| Stearalkonium Hectorite | 0.50 |
| Acrylic copolymer | 1.17 |
| Acetyl Tributyl Citrate | 5.45 |
| D&C Red # 7C Calcium Lake | 0.40 |
| Mica | 3.04 |
| TOTAL | 100.00 |

The formulation designated "1" above displayed the following properties when compared to a conventional nail enamel.

| | **Red Frost shade** | | |
|---|---|---|---|
| | Conventional | Nano | Change% |
| Slip Angle Test | 15 | 10 | -33.3 |
| Flexibility | OK | OK | OK |
| Adhesion (5=best: 1=worst) (cross hatch on glass) | 4 | 5 | |
| Mechanical Cupping Tests | 6.0 | 7.5 | +25.0 |
| Variable Height Impact Tester | 7.5 | 10.0 | +33.3 |
| Gloss (60 deg GM) | 50.9 | 62.86 | +23.5 |

The lower Slip Angle Test value indicates that a smaller angle was required before slip of a standard puck down the angle of incline in the nail enamel made with the nanoparticles as compared to nail enamel made without nanoparticles. The formulation designated "2" above displayed the following properties when compared to a conventional nail enamel.

| | **Red Frost shade** | | |
|---|---|---|---|
| | Conventional | Nano | Change% |
| Slip Angle Test | 20 | 10 | -50.0 |
| Flexibility | OK | OK | OK |
| Adhesion (5=best: 1=worst) (cross hatch on glass) | 3 | 5 | |
| Mechanical Cupping Tests | 6.8 | 7.7 | +13.24 |
| Variable Height Impact Tester | 10.0 | 12.5 | +25 |
| Gloss (60 deg GM) | 77.2 | 87.2 | +13.0 |

The lower Slip Angle Test value indicates that a smaller angle was required before slip of a standard puck down the angle of incline in the nail enamel made with the nanoparticles as compared to nail enamel made without nanoparticles.

The embodiments are described in sufficient detail to enable those skilled in the art to practice the invention. Other embodiments may be utilized and formulation and method of using changes may be made without departing from the scope of the invention. The detailed description is not to be taken in a limiting sense, and the scope of the invention is defined only by the appended claims.

## Claims

1. A mammalian nail enamel, comprising:
at least one film forming component;
a pigment for coloring the nail enamel wherein the pigment comprises nanoparticles;
at least one plasticizer;
at least one solvent; and
nanoparticles comprising nanoclay.

2. The nail enamel of claim 1, further comprising particles of one or more of clay, silica, or ceramic, wherein the particles are larger than nanoparticles.

3. The nail enamel of claim 1, wherein the nanoparticles comprising nanoclay have a concentration of 1 percent or less by weight of the nail enamel.

4. The nail enamel of claim 2, wherein the nanoparticles comprising nanoclay have a concentration within a range of about 0.05 to 0.5 percent by weight of the nail enamel.

5. The nail enamel of claim 4, wherein the particles larger than nanoparticles have a concentration within a range of about 0.05 to 0.5 percent by weight.

6. The nail enamel of claim 1, wherein the nanoparticles comprising nanoclay are coated with ammonium-based material such as dimethyl, dehydrogenated tallow, quaternary ammonium salt.

7. The nail enamel of claim 1, wherein the nanoparticles comprising nanoclay comprise particles having a 1- to 2-layer clay mineral with a plate-like structure, the platelet having a thickness of about one billionth of a meter and surface dimensions within a range of about 70 to more than 150 nanometers.

8. The nail enamel of claim 1, wherein the particle size of 90 percent of the nanoparticles comprising nanoclay, is less than 13 microns.

9. A base coat or a top coat or comprising the nail enamel of claim 1.

## Patentansprüche

1. Säugetiernagellack, umfassend:
mindestens eine filmbildende Komponente;
ein Pigment zum Färben des Nagellacks, wobei das Pigment Nanopartikel umfasst;
mindestens einen Weichmacher;
mindestens ein Lösungsmittel; und
Nanopartikel, die Nanoton umfassen.

2. Nagellack nach Anspruch 1, ferner umfassend Partikel aus einem oder mehreren von Ton, Siliciumdioxid oder Keramik, wobei die Partikel größer als Nanopartikel sind.

3. Nagellack nach Anspruch 1, wobei die Nanopartikel, die Nanoton umfassen, eine Konzentration von 1 Gewichtsprozent oder weniger des Nagellacks aufweisen.

4. Nagellack nach Anspruch 2, wobei die Nanopartikel, die Nanoton umfassen, eine Konzentration im Bereich von etwa 0,05 bis 0,5 Gewichtsprozent des Nagellacks aufweisen.

5. Nagellack nach Anspruch 4, wobei die Partikel, die größer als Nanopartikel sind, eine Konzentration im Bereich von etwa 0,05 bis 0,5 Gewichtsprozent aufweisen.

6. Nagellack nach Anspruch 1, wobei die Nanopartikel, die Nanoton umfassen, mit Material auf Ammoniumbasis beschichtet sind, wie etwa Dimethyl, dehydriertem Talg, quaternärem Ammoniumsalz.

7. Nagellack nach Anspruch 1, wobei die Nanopartikel, die Nanoton umfassen, Partikel umfassen, die ein 1- bis 2-lagiges Tonmineral mit einer plattenartigen Struktur aufweisen, wobei das Plättchen eine Dicke von etwa einem Milliardstel eines Meters und Oberflächenabmessungen in einem Bereich von etwa 70 bis mehr als 150 Nanometern aufweist.

8. Nagellack nach Anspruch 1, wobei die Partikelgröße von 90 % der Nanopartikel, die Nanoton umfassen, weniger als 13 Mikrometer beträgt.

9. Grundbeschichtung oder Deckschicht oder den Nagellack nach Anspruch 1 umfassend.

## Revendications

1. Vernis à ongles pour mammifère, comprenant :
au moins un composant filmogène ;
un pigment pour colorer le vernis à ongles dans lequel le pigment comprend des nanoparticules ;
au moins un plastifiant ;
et au moins un solvant ; et
des nanoparticules comprenant la nanoargile.

2. Vernis à ongles selon la revendication 1, comprenant également des particules d'un ou de plusieurs de l'argile, de la silice ou de la céramique, dans lequel les particules sont plus grandes que les nanoparticules.

3. Vernis à ongles selon la revendication 1, dans lequel les nanoparticules comprenant la nanoargile ont une concentration de 1 % ou moins en poids de l'émail pour ongle.

4. Vernis à ongles selon la revendication 2, dans lequel les nanoparticules comprenant la nanoargile ont une concentration dans une fourchette d'environ 0,05 à 0,5 % en poids du vernis à ongles.

5. Vernis à ongles selon la revendication 4, dans lequel les particules plus grandes que les nanoparticules ont une concentration dans une fourchette d'environ 0,05 à 0,5 % en poids.

6. Vernis à ongles selon la revendication 1, dans lequel les nanoparticules comprenant la nanoargile sont recouvertes d'un matériau à base d'ammonium tel que le diméthyle, le suif déshydrogéné ou un sel d'ammonium quaternaire.

7. Vernis à ongles selon la revendication 1, dans lequel les nanoparticules comprenant la nanoargile comprennent des particules ayant un minerai d'argile en 1 ou 2 couches avec une structure de type plaque, la plaquette ayant une épaisseur d'environ un milliardième de mètre et des dimensions de surface dans une fourchette d'environ 70 jusqu'à plus de 150 nm.

8. Vernis à ongles selon la revendication 1, dans lequel la taille de particule de 90 % des nanoparticules comprenant la nanoargile est inférieure à 13 microns.

9. Couche de base ou couche supérieure ou comprenant le vernis à ongles selon la revendication 1.
